Europäisches Patentamt
European Patent Office
Office européen des brevets

(19) (11) Veröffentlichungsnummer: **0 009 174**
**A1**

(12) # EUROPÄISCHE PATENTANMELDUNG

(21) Anmeldenummer: **79103291.5**

(22) Anmeldetag: **05.09.79**

(51) Int. Cl.³: **C 07 D 401/02**, A 61 K 31/37 // (C07D401/02, 217/14, 235/32)

(30) Priorität: **20.09.78 DE 2840927**

(43) Veröffentlichungstag der Anmeldung: **02.04.80 Patentblatt 80/7**

(84) Benannte Vertragsstaaten: **AT BE CH DE FR GB IT NL SE**

(71) Anmelder: **BAYER Aktiengesellschaft, Zentralbereich Patente,Marken und Lizenzen Bayerwerk, D-5090 Leverkusen 1 (DE)**

(72) Erfinder: **Niemers, Ekkehard, Dr., In den Birken 51a, D-5600 Wuppertal-1 (DE)**
Erfinder: **Thomas, Herbert, Dr., Bergerheide 82b, D-5600 Wuppertal-1 (DE)**

(54) **Neue (5-(3,4-Dihydroisochinolin-1-yl)-1H-benzimidazol-2-yl)-carbaminsäurealkylester, Verfahren zu ihrer Herstellung, sie enthaltende Arzneimittel und Verfahren zur Herstellung von anthelmintisch wirksamen Mitteln.**

(57) Die neuen Titelverbindungen der allgemeinen Formel

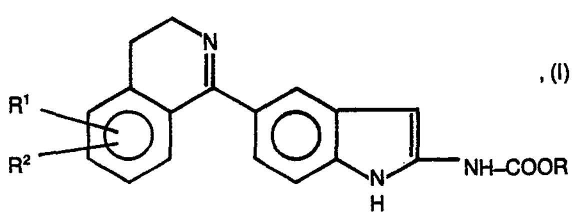

, (I)

in welcher R für Alkyl steht, $R^1$ und $R^2$ gleich oder verschieden sein können und für Wasserstoff, Halogen, Alkyl, Halogenalkyl, Alkoxy, Alkylthio, Alkoxyalkyl, Halogenalkoxy oder Halogenalkylthio stehen, z. B. die Verbindung [5-(3,4-Dihydroisochinolin-1-yl)-1H-benzimidazol-2-yl]-carbaminsäuremethylester, sowie ihre physiologisch verträglichen Salze besitzen ausgeprägte anthelmintische Wirksamkeit in der Human- wie in der Veterinärmedizin. Sie werden erhalten, indem man die – in ihrer Herstellung beschriebenen – neuen substituierten o-Phenylendiamine der allgemeinen Formel

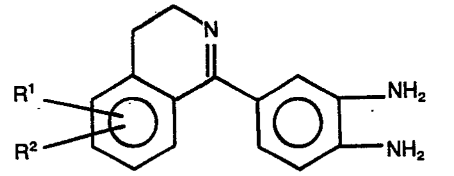

., (II)

in einem Lösungsmittel bzw. Lösungsmittelgemisch bei Temperaturen von ca. 0 bis 120°C mit einem Kohlensäure-Derivat, insbesondere N,N'-bis- Methoxycarbonyl-isothioharnstoff-S-methyläther der Formel

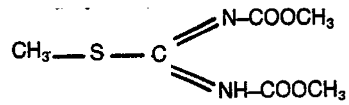

zweckmäßig in Gegenwart einer Säure, umsetzt.

EP 0 009 174 A1

BAYER AKTIENGESELLSCHAFT 5090 Leverkusen, Bayerwerk
Zentralbereich
Patente, Marken und Lizenzen Er-by

BEZEICHNUNG GEÄNDERT
siehe Titelseite

3,4-Dihydroisochinoline, Verfahren zu ihrer Herstellung sowie ihre Verwendung als Arzneimittel

Die vorliegende Erfindung betrifft neue 3,4-Dihydroisochinoline, Verfahren zu ihrer Herstellung und ihre Verwendung als Arzneimittel, insbesondere als Anthelmintika.

Es ist bereits bekanntgeworden, daß Benzimidazole der allgemeinen Formel

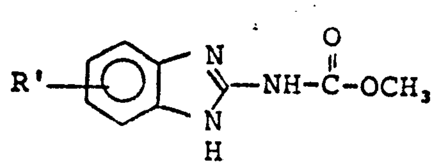

in der

R' Alkylthio, Arylthio oder Benzoyl bedeuten kann,

anthelmintische Wirkungen aufweisen (siehe dazu Deutsche Offenlegungsschriften 2 527 065, 2 250 469 und 2 246 605).

Die Wirksamkeit der in den vorgenannten Deutschen Offenlegungsschriften genannten Wirkstoffe ist jedoch derjenigen der im folgenden charakterisierten erfindungsgemäßen Wirkstoffe eindeutig unterlegen.

Durch vorliegende Erfindung werden neue 3,4-Dihydroisochinoline der Formel

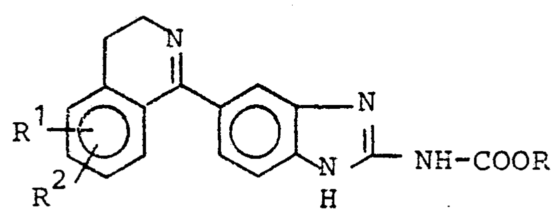

(I)

und ihre physiologisch verträglichen Salze bereitgestellt, wobei

R für Alkyl steht,

$R^1$ und $R^2$ gleich oder verschieden sein können und für Wasserstoff, Halogen, Alkyl, Halogenalkyl, Alkoxy, Alkylthio, Alkoxyalkyl, Halogenalkoxy oder Halogenalkylthio stehen.

Die erfindungsgemäßen Wirkstoffe zeigen eine besonders stark ausgeprägte anthelmintische Wirksamkeit.

Besonders bevorzugt sind Verbindungen der allgemeinen Formel (I) in welcher

R für Alkyl ($C_1$-$C_6$) steht ,

$R^1$ und $R^2$ gleich oder verschieden sein können und für Wasserstoff, Fluor, Chlor, Brom, Alkyl ($C_1$-$C_6$), Trifluormethyl, Alkoxy ($C_1$-$C_6$), Trifluormethoxy oder Trifluormethylthio stehen.

Die erfindungsgemäßen Wirkstoffe der obigen Formel (I) können hergestellt werden, indem man in einem geeigneten Lösungsmittel bzw. Lösungsmittelgemisch bei Temperaturen von ca. 0 bis ca. 120°C substituierte ortho-Phenylendiamine der Formel

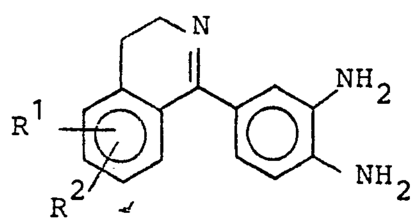

(II)

in welcher

$R^1$ und $R^2$ die bei Formel (I) angegebene Bedeutung haben,

mit einem Kohlensäurederivat der Formel

a) 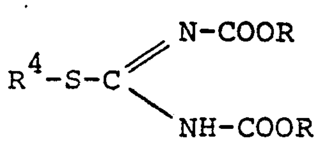

(III)

oder

b) $H_3C-O-\overset{\overset{NH}{\|}}{C}-NH-COOR$ (IV)

oder

c) $SCN-\overset{\overset{O}{\|}}{C}-OR$ (V)

oder

d) $R^4-S-\overset{\overset{NH}{\|}}{C}-NH-COOR$ (VI)

oder

e) 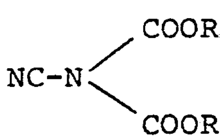 (VII)

oder

f) $NC-NH-COOR$ (VIII)

wobei

R die oben angegebene Bedeutung besitzt und

$R^4$ für Alkyl ($C_1$-$C_4$) steht,

gegebenenfalls in Gegenwart einer Säure umsetzt und die erhaltenen Reaktionsprodukte nach üblichen Methoden aufarbeitet.

Die einzelnen Verfahrensvarianten zur Herstellung der erfindungsgemäßen Verbindungen können durch die folgenden schematischen Darstellungen wiedergegeben werden, wobei im folgenden $R^I$ für den Substituenten

steht und 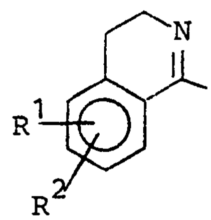

wobei

$R^1$ und $R^2$ die bei Formel (I) oben angegebenen Bedeutungen besitzen:

a) 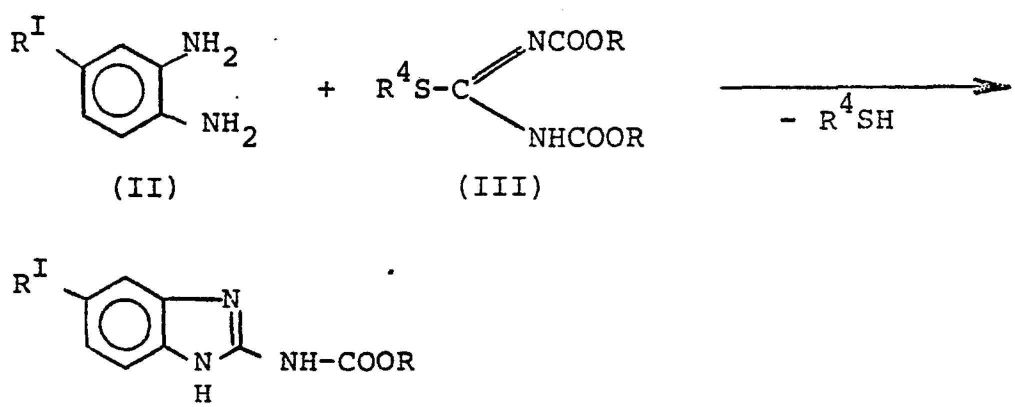


(II) (III)

(I)

b)

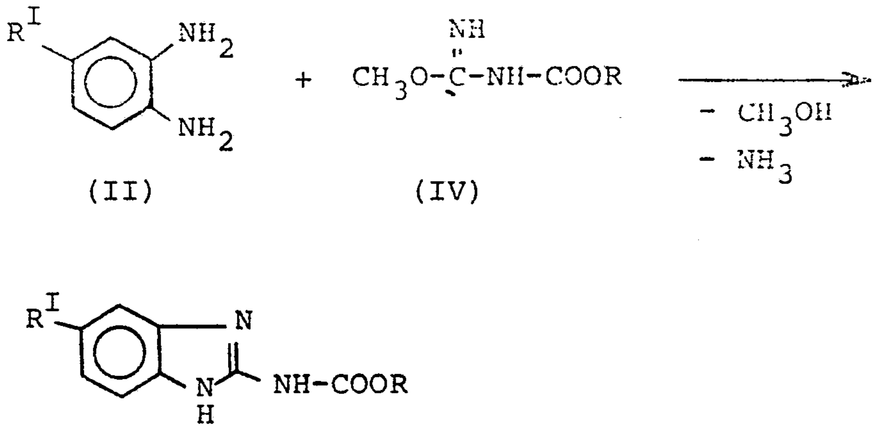


(II) (IV)

(I)

c)

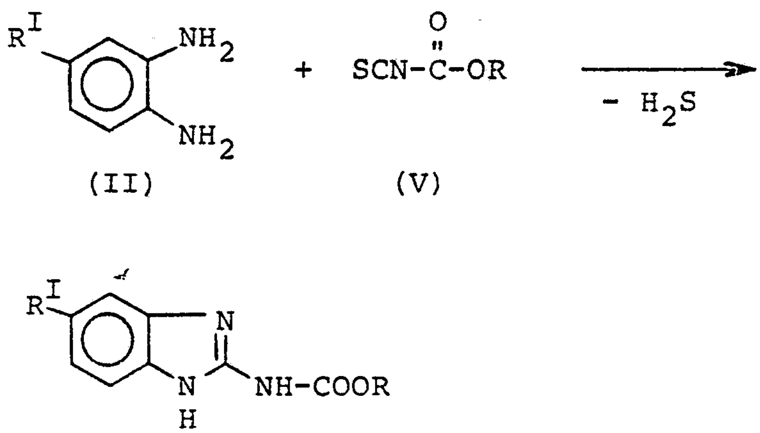


(II) (V)

(I)

d)

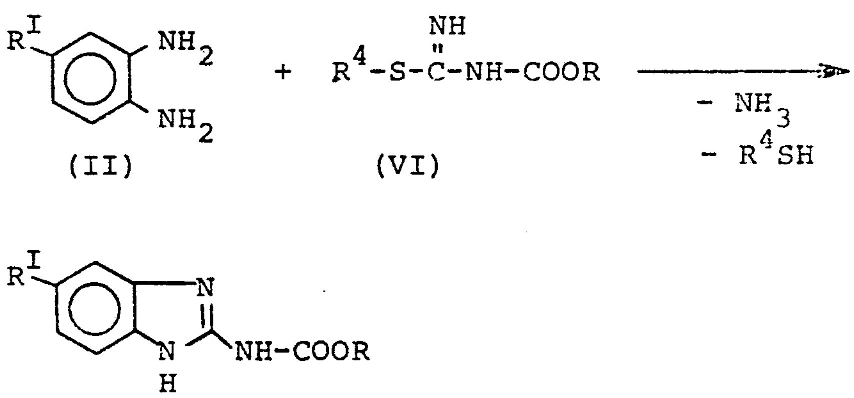


(II) (VI)

(I)

e) 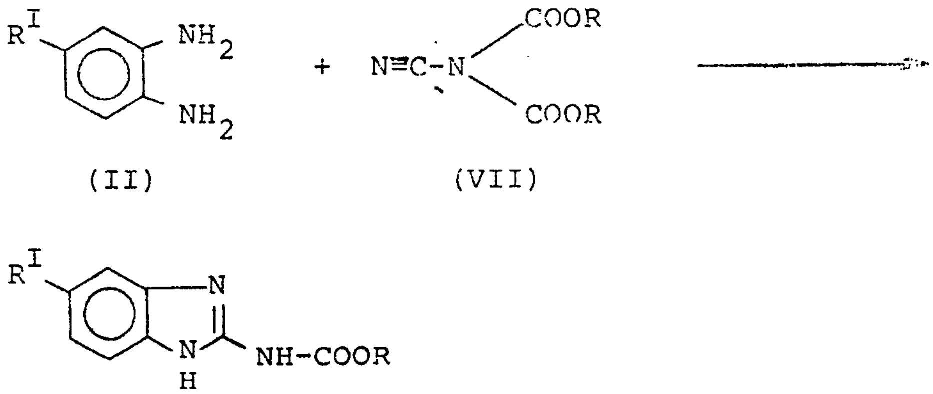

(II) (VII)

f) 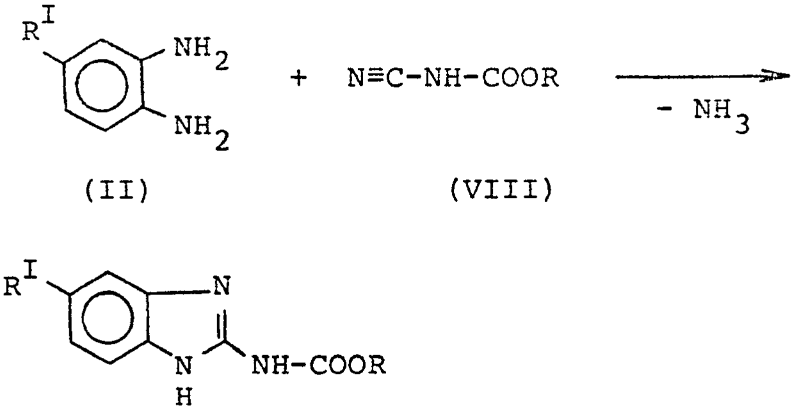


(II) (VIII)

und wobei die Substituenten R und $R^4$ die oben angegebene Bedeutung besitzen können.

Als R, $R^1$ bzw. $R^2$ = Alkyl der Formeln (I), (III), (IV), (V), (VI), (VII) und (VIII) steht vorzugsweise Alkyl ($C_1$-$C_6$) und insbesondere Alkyl ($C_1$-$C_4$). Beispielhaft seien aufgeführt: Methyl, Äthyl, n-Propyl, i-Propyl, n-Butyl, i-Butyl, t-Butyl, n-Pentyl, n-Hexyl.

Als Halogen $R^1$ bzw. $R^2$ der Formeln (I) und (II) stehen vorzugsweise Fluor, Chlor, Brom und Jod, insbesondere Fluor, Chlor und Brom.

Halogenalkyl $R^1$ und $R^2$ der Formeln (I) und (II) enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlor-di-fluormethyl, Brommethyl, 2,2,2-Trifluoräthyl und Pentafluoräthyl genannt. Als besonders bevorzugt sei Trifluormethyl aufgeführt.

Als $R^1$ und $R^2$ = Alkoxy der Formeln (I) und (II) steht geradkettiges oder verzweigtes Alkoxy mit vorzugsweise 1 bis 6, insbesondere 1 bis 4 Kohlenstoffatomen. Beispielhaft seien Methoxy, Äthoxy, n- und i-Propoxy und n-, i- und t-Butoxy genannt.

Als $R^1$ und $R^2$ = Alkylthio der Formeln (I) und (II) steht geradkettiges oder verzweigtes Alkylthio mit vorzugsweise 1 bis 6, insbesondere 1 bis 4 Kohlenstoffatomen. Beispielhaft seien Methylthio, Äthylthio, n- und i-Propylthio, n-, i- und t-Butylthio genannt.

Als Alkoxyalkyl $R^1$ und $R^2$ der Formeln (I) und (II) steht geradkettiges oder verzweigtes Alkoxyalkyl mit vorzugsweise 1 bis 6, insbesondere 1 bis 4 Kohlenstoffatomen

im Alkylteil und vorzugsweise 1 bis 6, insbesondere 1 bis 4 Kohlenstoffatome im Alkoxyteil. Beispielhaft seien Methoxymethyl, Äthoxymethyl, n-Propoxymethyl, n-Butoxymethyl, Äthoxyäthyl, Methoxyäthyl genannt.

Als Halogenalkoxy $R^1$ und $R^2$ der Formeln (I) und (II) kommt geradkettiges oder verzweigtes Halogenalkoxy mit vorzugsweise 1 bis 6, insbesondere 1 bis 4 Kohlenstoffatomen in Frage, als Halogensubstituenten kommen vorzugsweise Fluor und Chlor in Frage. Beispielhaft seien genannt: Trifluormethoxy, Trifluoräthoxy, Trichlormethoxy, Pentafluoräthoxy. Besonders bevorzugt ist Trifluormethoxy.

Als Halogenalkylthio $R^1$ und $R^2$ der Formeln (I) und (II) kommt geradkettiges oder verzweigtes Halogenalkylthio mit vorzugsweise 1 bis 6, insbesondere 1 bis 4 Kohlenstoffatomen in Frage, als Halogensubstituenten kommen vorzugsweise Fluor und Chlor in Frage. Beispielhaft seien genannt: Trifluormethylthio, Trifluoräthylthio, Trichlormethylthio, Pentafluoräthylthio. Besonders bevorzugt ist Trifluormethylthio.

Die als Ausgangsstoffe verwendeten substituierten o-Phenylendiamine der Formel (II) sind noch nicht bekannt. Sie können aber in an sich bekannter Weise nach folgendem Reaktionsschema hergestellt werden:

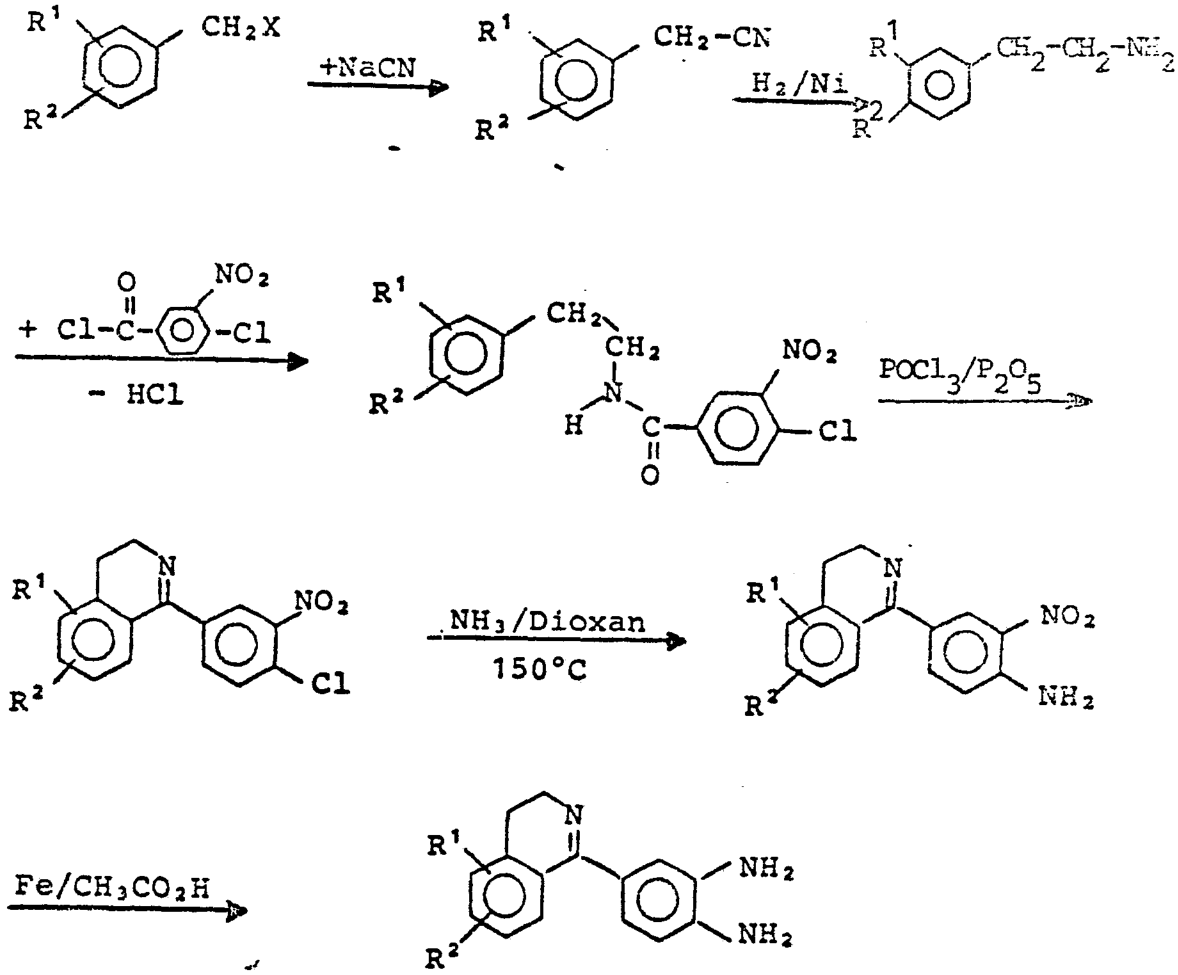


(II)

wobei in der Ausgangskomponente der Substituent X für Halogen, vorzugsweise für Chlor oder Brom steht.

Für die Reaktionsvarianten a) bis f) gilt:

Als Lösungsmittel bzw. Lösungsmittelgemische kommen alle polaren organischen Lösungsmittel in Frage. Hierzu gehören vorzugsweise Alkohole, wie Methanol, Äthanol, iso-

Propanol, sowie deren Gemische mit Wasser, Ketone wie Aceton (auch mit Wasser gemischt), aber auch Äther wie Dioxan oder Tetrahydrofuran und Chlorkohlenwasserstoffe wie Chloroform, Methylenchlorid oder Chlorbenzol.

Die als reaktionsfördernde Katalysatoren zugesetzten Säuren können im Prinzip aus der Reihe der bekannten organischen oder anorganischen Säuren beliebig ausgewählt werden. Vorteilhaft verwendet man jedoch die leicht zugänglichen, technisch bedeutenden Vertreter dieser Klassen. Als Beispiele seien genannt: Salzsäure, Schwefelsäure, Salpetersäure, Ameisensäure, Essigsäure, p-Toluolsulfonsäure.

Die Reaktionstemperaturen können in einem größeren Bereich variiert werden. Im allgemeinen arbeitet man zwischen 0°C und 120°C, vorzugsweise zwischen 20°C und 100°C. Die Umsetzung wird im allgemeinen bei Normaldruck durchgeführt. Bei der Durchführung der Umsetzungen gemäß den Verfahrensvarianten a) bis f) werden jeweils vorzugsweise äquimolekulare Mengen der Reaktionspartner eingesetzt. Es kann aber durchaus einer der Reaktionspartner in einem Überschuß zugesetzt werden.

Die Aufarbeitung der Reaktionsprodukte erfolgt nach den in der präparativen organischen Chemie üblichen Methoden.

Die Herstellung der physiologisch verträglichen Salze kann aus den gereinigten und gegebenenfalls bereits umkristallisierten Verbindungen der Formel (I) durch Zugabe einer entsprechenden Menge einer geeigneten Säure oder in Lösung durch Ausfällen der Salze in organischen Lösungsmitteln und Auswaschen dieser Salze mit inerten Lösungsmitteln nach an sich bekannten Methoden geschehen. Als Säuren kommen z.B. in Frage: Salzsäure, Schwefelsäure, Phosphorsäure, Salpetersäure, Maleinsäure, Fumarsäure, Essigsäure, Methansulfonsäure, Naphthalindisulfonsäure.

Die neuen Verbindungen sind oral und parenteral applizierbar, wobei sich auch in Form ihrer physiologisch verträglichen Salze wie beispielsweise Hydrohalogenide - vorzugsweise Hydrochloride-, Sulfate, Phosphate, Nitrate, Maleinate, Fumarate, Acetate, Methansulfonate, Naphthalindisulfonate u.a.m. angewendet werden.

Die erfindungsgemäßen Verbindungen zeigen eine überraschend gute und breite Wirkung in einem Bereich von 0,1 bis 20 mg/kg Körpergewicht bei verschiedenen Tierarten gegen folgende Nematoden und Cestoden:

1. Hakenwürmer (z.B. Uncinaria stenocephala, Ancylostoma caninum, Bunostomun trigonocephalum)

2. Trischostrongyliden (z.B. Nippostrongylus muris, Haemonchus contortus, Trichostrongylus colubriformis, Ostertagia circumcincta),

3. Strongyliden (z.B. Oesophagostomum columbianum),

4. Rhabditiden (z.B. Strongyloides ratti),

5. Spulwürmer (z.B. Ascaris suum, Toxocara canis, Toxascaris leonina),

6. Madenwürmer (z.B. Aspiculuris tetraptera),

7. Heterakiden (z.B. Heterakis spumosa),

8. Peitschenwürmer (z.B. Trichuris muris),

9. Filarien (z.B. Litomosoides carinii, Dipetalonema witei),

10. Cestoden (z.B. Hymenolepis nana, Taenia pisiformis, Echinococcus multilocularis),

11. Trematoden (z.B. Fasciola hepatica).

Die Wirkung wurde im Tierversuch nach oraler und parenteraler Applikation bei stark mit Parasiten befallenen Versuchstieren geprüft. Die angewendeten Dosierungen wurden sehr gut von den Versuchstieren vertragen.

Die neuen Wirkstoffe können als Anthelmintika sowohl in der Human- als auch in der Veterinärmedizin verwendet werden.

Die neuen Wirkstoffe können in bekannter Weise in die üblichen Formulierungen übergeführt werden.

Die neuen Verbindungen können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine orale Verabreichung in Betracht kommenden Tabletten und dergleichen mit Süßstoffzusatz und ähnlichem versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um den obengenannten Dosierungspielraum zu erreichen.

Die Formulierungen werden in bekannter Weise hergestellt, z.B. durch Verstrecken der Wirkstoffe mit Lösungsmitteln

und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nicht toxische organische Lösungsmittel, wie Paraffine (z.B. Erdölfraktionen), pflanzliche Öle (z.B. Erdnuß-/Sesamöl), Alkohole (z.B. Äthylalkohol, Glycerin), Glykole (z.B.Propylenglykol, Polyäthylenglykol) und Wasser; feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker); Emulgiermittel, wie nicht ionogene und anionische Emulgatoren (z.B. Polyoxyäthylen-Fettsäure-Ester, Polyoxyäthylen-Fettalkohol-Äther, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze, wie Natriumcitrat, Calciumcarbonat und Dicalciumphosphat, zusammen mit verschiedenen Zuschlagstoffen, wie Stärke, vorzugsweise Kartoffelstärke, Gelatine und dergleichen, enthalten. Weiterhin können

Gleitmittel, wie Magnesiumstearat, Natriumlaurylsulfat und Talkum, zum Tablettieren mitverwendet werden.

Im Falle wäßriger Suspensionen und/oder Elixieren, die für orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Wirkstoffe können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die neuen Verbindungen können in den Formulierungen auch in Mischungen mit anderen bekannten Wirkstoffen vorliegen.

Die neuen Wirkstoffe können in üblicher Weise angewendet werden. Die Applikation erfolgt vorzugsweise oral beziehungsweise subkutan, eine dermale Applikation ist jedoch ebenfalls möglich.

Im allgemeinen hat es sich als vorteilhaft erwiesen, Mengen von etwa 0,1 bis etwa 50 mg der neuen Verbindungen je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Human- und Veterinärmedizin ist der gleiche Dosierungsspielraum vorgesehen. Sinngemäß gelten auch die weiteren obigen Ausführungen.

Herstellungsbeispiele

Beispiel 1

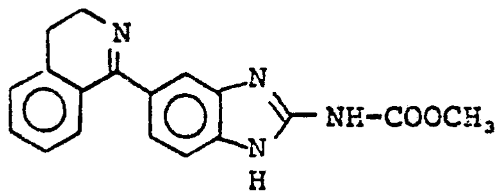

Ein Gemisch von 23,7 g (0,1 Mol) 1-(3,4-Diaminophenyl)-3,4-dihydroisochinolin, 22 g (0,1 Mol) N,N'-bis-Methoxycarbonyl-isothioharnstoff-S-methyläther, 16,5 ml Eisessig, 300 ml Äthanol und 300 ml Wasser wird 7 h unter Rückfluß gekocht. Nach dem Erkalten wird das Gemisch durch Zugabe von Ammoniak schwach alkalisch gestellt. Man saugt den erhaltenen Niederschlag ab und wäscht mit Äthanol und Äther nach. Es werden 27,8 g (84 % d.Th.) /5-(3,4-Dihydroisochinolin-1-yl)-1H-bemzimidazol-2-yl/-carbaminsäuremethylester vom Fp. 250°C (Zers.) erhalten.

Vorprodukte:

Das als Ausgangsmaterial verwendete 1-(3,4-Diaminophenyl)-3,4-dihydroisochinolin (Fp. 231-233°C) wird durch Reduktion von 1-(4-Amino-3-nitrophenyl)-3,4-dihydroisochinolin mit Eisen in verdünnter Essigsäure (als Lösungsmittel) erhalten.

1-(4-Amino-3-nitrophenyl)-3,4-dihydroisochinolin (Fp. 194-196°C) wird durch Umsetzung von 1-(4-Chlor-3-nitrophenyl)-3,4-dihydroisochinolin mit Ammoniak bei 150°C im Autoklaven erhalten.

1-(4-Chlor-3-nitrophenyl)-3,4-dihydroisochinolin (Fp. 74-76°C) wird durch Erhitzen von 4-Chlor-3-nitro-N-phenyläthylbenzamid in einem Gemisch aus Phosphoroxychlorid und Phosphorpentoxid auf 130°C erhalten.

4-Chlor-3-nitro-N-phenyläthylbenzamid (Fp. 95-96°C) wird durch Umsetzung von 4-Chlor-3-nitrobenzoesäurechlorid mit Phenäthylamin erhalten.

Beispiel 2

Nach der in Beispiel 1 beschriebenen Methode erhält man aus N,N'-bis-Methoxycarbonyl-S-methyl-isothioharnstoff und substituierten 1-(3,4-Diaminophenyl)-3,4-dihydroisochinolinen folgende Verbindungen:

| Ausgangsstoffe | | | Erfindungsgemäß hergestellte Stoffe | | | |
|---|---|---|---|---|---|---|
| 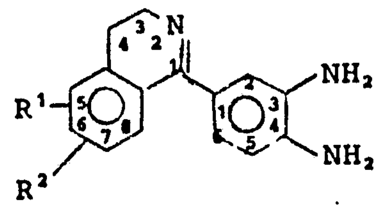 | | | 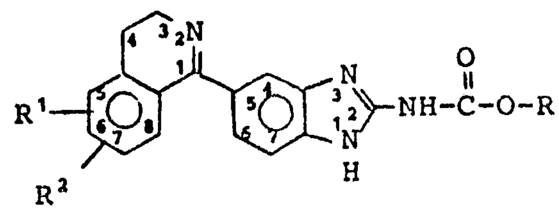 | | | |
| $R^1$ | $R^2$ | Fp(°C) | $R^1$ | $R^2$ | R | Fp(°C) |
| 5-Cl | H | 182-183°C | 5-Cl | H | $CH_3$ | 240°C (Zers.) |
| 6-Cl | H | | 6-Cl | H | $CH_3$ | |
| 7-Cl | H | 133-135°C | 7-Cl | H | $CH_3$ | 255°C (Zers.), |
| 5-F | H | | 5-F | H | $CH_3$ | |
| 6-F | H | | 6-F | H | $CH_3$ | |
| 7-F | H | 230-232°C | 7-F | H | $CH_3$ | 250°C (Zers.) |
| 5-$CF_3$ | H | | 5-$CF_3$ | H | $CH_3$ | |
| 6-$CF_3$ | H | | 6-$CF_3$ | H | $CH_3$ | |
| 7-$CF_3$ | H | | 7-$CF_3$ | H | $CH_3$ | |
| 5-$CH_3$ | H | | 5-$CH_3$ | H | $CH_3$ | |
| 6-$CH_3$ | H | | 6-$CH_3$ | H | $CH_3$ | |
| 7-$CH_3$ | H | 139-141°C | 7-$CH_3$ | H | $CH_3$ | 250°C (Zers.) |
| 5-Cl | 6-Cl | | 5-Cl | 6-Cl | $CH_3$ | |
| 5-Cl | 7-Cl | | 5-Cl | 7-Cl | $CH_3$ | |
| 6-Cl | 7-Cl | | 6-Cl | 7-Cl | $CH_3$ | |

| $R^1$ | $R^2$ | Fp. (°C) | $R^1$ | $R^2$ | R | Fp. (°C) |
|---|---|---|---|---|---|---|
| 5-F | 7-F | | 5-F | 7-F | $CH_3$ | |
| 7-$OCF_3$ | H | | 7-$OCF_3$ | H | $CH_3$ | |
| 7-$SCF_3$ | H | | 7-$SCF_3$ | H | $CH_3$ | |
| 5-$OCH_3$ | H | | 5-$OCH_3$ | H | $CH_3$ | |
| 6-$OCH_3$ | H | 183-184°C | 6-$OCH_3$ | H | $CH_3$ | 245°C (Zers.) |
| 7-$OCH_3$ | H | 106-107°C | 7-$OCH_3$ | H | $CH_3$ | 230°C (Zers.) |
| 6-$OCH_3$ | 7-$OCH_3$ | | 6-$OCH_3$ | 7-$OCH_3$ | $CH_3$ | |
| 6-$OCH_3$ | 8-$OCH_3$ | | 6-$OCH_3$ | 8-$OCH_3$ | $CH_3$ | |
| H | H | 231-233°C | H | H | $C_2H_5$ | |
| 7-Cl | H | 133-135°C | 7-Cl | H | $C_2H_5$ | |
| 7-F | H | 230-232°C | 7-F | H | $C_2H_5$ | |
| 7-F | H | 230-232°C | 7-F | H | i-$C_3H_7$ | |
| 7-Cl | H | 133-135°C | 7-Cl | H | n-$C_4H_9$ | |
| H | H | 231-233°C | H | H | n-$C_6H_{13}$ | |

Patentansprüche

1. 3,4-Dihydroisochinoline der allgemeinen Formel

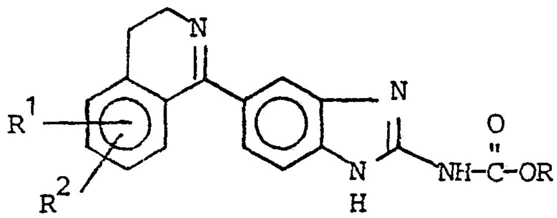

(I)

in welcher

R für Alkyl steht,

$R^1$ und $R^2$ gleich oder verschieden sein können und für Wasserstoff, Halogen, Alkyl, Halogenalkyl, Alkoxy, Alkylthio, Alkoxyalkyl, Halogenalkoxy oder Halogenalkylthio stehen,

sowie deren physiologisch verträglichen Salze.

2. 3,4-Dihydroisochinoline der Formel (I) gemäß Anspruch 1 in welcher

R für Alkyl ($C_1$-$C_6$) steht

$R^1$ und $R^2$ gleich oder verschieden sein können und für Wasserstoff, Fluor, Chlor, Brom, Alkyl ($C_1$-$C_6$), Trifluormethyl, Alkoxy ($C_1$-$C_6$), Trifluormethoxy oder Trifluormethylthio stehen

sowie deren physiologisch verträglichen Salze.

3. Verfahren zur Herstellung von 3,4-Dihydroisochinolinen der allgemeinen Formel

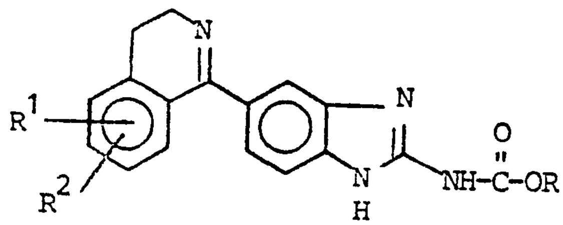

(I)

in welcher

R für Alkyl steht

$R^1$ und $R^2$ gleich oder verschieden sein können und für Wasserstoff, Halogen, Alkyl, Halogenalkyl, Alkoxy, Alkoxyalkyl, Halogenalkoxy oder Halogenalkylthio stehen,

dadurch gekennzeichnet, daß man in einem geeigneten Lösungsmittel bzw. Lösungsmittelgemisch bei Temperaturen von ca. 0 bis ca. 120°C, substituierte ortho-Phenylendiamine der Formel

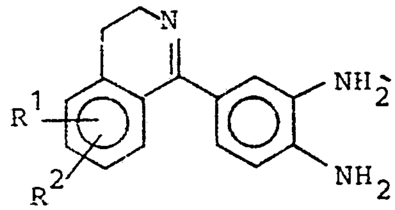

(II)

mit einem Carbonsäurederivat der Formel

a) 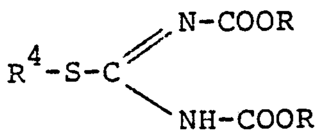

(III)

oder

b) 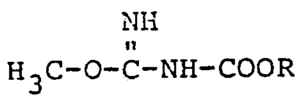

(IV)

oder

c) 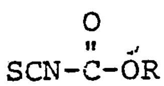

(V)

oder

d) 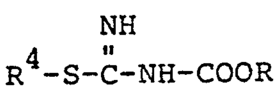

(VI)

oder

e) 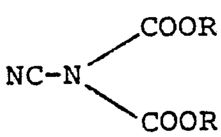

(VII)

oder

f) NC-NH-COOR (VIII)

wobei

R die oben angegebene Bedeutung besitzt und

$R^4$ für Alkyl ($C_1$-$C_4$) steht,

gegebenenfalls in Gegenwart einer Säure umsetzt und die erhaltenen Reaktionsprodukte nach üblichen Methoden aufarbeitet.

4. Arzneimittel, gekennzeichnet durch einen Gehalt an mindestens einem 3,4-Dihydroisochinolin gemäß Anspruch 1.

5. Arzneimittel, gekennzeichnet durch einen Gehalt an mindestens einem 3,4-Dihydroisochinolin gemäß Anspruch 2.

6. Verfahren zur Herstellung von anthelmintisch wirksamen Mitteln, dadurch gekennzeichnet, daß man 3,4-Dihydroisochinoline gemäß Anspruch 1 mit inerten nichttoxischen, pharmazeutisch geeigneten Trägerstoffen vermischt.

7. Verwendung von Verbindungen gemäß Anspruch 1 bei der Bekämpfung von Krankheiten.

8. Verwendung von Verbindungen gemäß Anspruch 1 bei der Bekämpfung von Helminthiasen.

9. Verfahren zur Behandlung von Helminthiasen, dadurch gekennzeichnet, daß man 3,4-Dihydroisochinoline gemäß Anspruch 1 Menschen oder Tieren appliziert, die an Helminthiasen erkrankt sind.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0009174

Nummer der Anmeldung

EP 79103291.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A1 - 2 527 065 (SMITH KLINE CORPORATION)<br>+ Gesamtes Dokument +<br>-- | 1-6 |
| D | DE - A - 2 250 469 (HOECHST)<br>+ Gesamtes Dokument +<br>-- | 3 |
| D | DE - A - 2 246 605 (ICI)<br>+ Gesamtes Dokument +<br>-- | 3 |
| A | DE - A1 - 2 635 326 (F. HOFFMAN-LA ROCHE)<br>+ Beispiel 11 +<br>-- | 1-6 |
| A | GB - A - 1 123 317 (SMITH KLINE CORPORATION)<br>+ Gesamtes Dokument +<br>---- | 1-6 |

KLASSIFIKATION DER ANMELDUNG (Int.Cl. 3)

C 07 D 401/02
A 61 K 31/37//
(C 07 D 401/02, 217/14, 235/32)

RECHERCHIERTE SACHGEBIETE (Int. Cl. 3)

C 07 D 401/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6

Unvollständig recherchierte Patentansprüche:

Nicht recherchierte Patentansprüche: 7-9

Grund für die Beschränkung der Recherche: Verfahren zur therapeutischen Behandlung des menschlichen oder tierischen Körpers (Siehe Art. 52(4) des Europäischen Patentübereinkommens)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Wien | 31-10-1979 | DASCHL |